# EUROPÄISCHE PATENTSCHRIFT

(11) **EP 1 101 451 B1**
(45) Veröffentlichungstag und Bekanntmachung des Hinweises auf die Patenterteilung: **20.04.2005**
(21) Anmeldenummer: 00123977.1
(22) Anmeldetag: 03.11.2000
(51) Int. Cl.: A61C 1/08

(54) **Verfahren zur Herstellung einer Bohrhilfe für ein Zahnimplantat**
Method of making a drill guide for a dental implant
Procédé de fabrication d'un guide de perçage pour un implant dentaire

(30) Priorität: 03.11.1999 DE 19952962
(43) Veröffentlichungstag der Anmeldung: 23.05.2001
(73) Patentinhaber: Sirona Dental Systems GmbH, 64625 Bensheim (DE)
(72) Erfinder: Scherer, Franz, 50158 Köln (DE); Pfeiffer, Joachim, 64625 Bensheim (DE)
(74) Vertreter: Sommer, Peter

(56) Entgegenhaltungen:
- WO-A-99/32045
- DE-A- 19 510 294
- DE-A- 19 629 708
- US-A- 5 343 391
- US-A- 5 842 858
- US-A- 5 967 777

## Beschreibung

Die Erfindung bezieht sich auf ein Verfahren zur Herstellung einer Bohrhilfe zum exakten Anbringen des Führungsloches für ein Zahnimplantat, wobei das Führungsloch für das Implantat an noch im Kiefer vorhandenen Zähnen ausgerichtet wird.

WO 99/32045 offenbart ein Verfahren zur Herstellung einer dentalen Bohrhilfe für Implantate. Gemäß der in dieser Veröffentlichung vorgeschlagenen Vorgehensweise wird anhand eines Kieferbildes mit Bezug auf eine Abdruckfläche ein dreidimensionales Rechnerbild modelliert. Anhand der auf diese Weise erzeugten Computergraphik wird mindestens eine Bohrlochposition bestimmt, wobei deren Position in drei Dimensionen spezifiziert wird, einschließlich des Bohrlochendpunktes und der Bohrlochlänge in Bezug auf den Kieferabdruck. Danach wird mindestens ein Satz von Implantatbohrlochkoordinaten in eine computergesteuerte Präzisionswerkzeugmaschine eingespeist. An einem Bohrkörper ist eine erste Oberfläche vorgesehen, welche zu der Abdruckfläche des Kiefers korrespondiert. Mittels der Präzisionswerkzeugmaschine wird im Bohrkörper für jeden der zuvor eingespielten Bohrlochkoordinatensätze ein Bohrführungssockel vorbereitet mit entsprechend der anhand des Kieferabschnittes ermittelten Bohrlochposition und Bohrlochorientierung.

Gemäß dieser Methode wird die Bohrlochlage und die Bohrlochorientierung anhand eines vom Kieferknochen genommenen Abdruckes bestimmt. Beim Setzen von Zahnimplantaten werden die Form und die Größe des Implantates anhand von Röntgenaufnahmen genau geplant. Die Lage des Implantates im Kiefer wird möglichst genau vorherbestimmt. Dazu bedient man sich Bohrschablonen, die die Erzeugung eines präzise positionierten Bohrloches ermöglichen sollen. In der Regel ist es jedoch schwierig, die Position des Führungsloches beim Bohren genau zu bestimmen, da die auf den Röntgenbildern enthaltenen Informationen nicht exakt auf die optischen Bilder, die der Arzt beim Bohren sieht, übertragen werden können. Der Arzt verläßt sich auf seine Erfahrung, insbesondere was die Lage und den Verlauf des in den Kieferknochen verlaufenden Nervenstränge betrifft. So entsteht das unbefriedigende Ergebnis, daß zwar Implantate hochgenau geplant und gefertigt werden, ihre Positionierung im Kieferbereich aber bisher anhand von Erfahrungswerten erfolgt, die individuell stark variieren können.

Aus DE 197 25 197 A1 ist ein Verfahren und eine Vorrichtung zur Lokalisierung von Zahnimplantaten sowie eine Vorrichtung zur Koordinatenzuordnung bekannt geworden. Dieses Verfahren und die Vorrichtung zur Lokalisierung von Zahnimplantaten verarbeiten meßtechnisch erfaßte anatomische Daten bezüglich des Aufbaus eines Kieferknochens des betreffenden Patienten. Aufgrund der abgespeicherten Daten werden Schnittbildinformationen generiert, die zur Definition des Implantationsortes verwendet werden. Über ein zugeordnetes Bezugskoordinatensystem können die tatsächlichen Knochengeometrien, die meßtechnischen Informationen, die Gestaltung eines Kiefergipsmodells sowie die Geometrie einer Operationshilfsschablone derart exakt relativ zueinander in Bezug gesetzt werden, daß eine hochgenaue Plazierung einer Bohrung im Kieferknochen zur Aufnahme des Zahnimplantates unterstützt wird. Die Übertragung der jeweiligen Koordinateninformationen auf die verwendeten Hilfseinrichtungen und Bearbeitungseinrichtungen wird von einem mechanischen Übertragungselement unterstützt, das beispielsweise eine bogenförmige Ausbildung besitzen kann und einen in den Mundbereich des Patienten einführbaren Adapter beinhaltet.

Aus anderen Veröffentlichungen, zum Beispiel US 5,888,065 sind beispielsweise Herstellungsverfahren für Implantatbohrungen im Kiefer bekannt, bei denen vollständig auf eine genaue Bestimmung der Führungslochposition verzichtet wird.

Die US 5 967 777 schlägt eine Bohrschablone, die aus einem körperlich vorhandenen Modell des oder der zu implantierenden Zähne und Befestigungsmitteln besteht, sowie eine Methode zur Festlegung und Ausführung des Bohrloches vor. Die Bohrführung wird mit Hilfe einer computergesteuerten Fräsmaschine unter Vorlage eines aus einer CT-Aufnahme gewonnenen computergenerierten Modells des Kieferknochens in das körperlich vorhandene Modell des zu implantierenden Zahns gefräst.

US 5 842 858 offenbart ein Verfahren, das mindestens ein Bild eines Kiefers, welches aus mindestens einer Röntgenaufnahme oder einem anderen bildgebenden Verfahren (CT, Kernspintomographie) mit einer 3D-Lagebstimmung unter Zuhilfenahme von eindeutig am Kiefer platzierten Messpunkten korreliert und daraus die aktuelle, relative Lage des Kiefers zu einem Referenzpunkt bestimmt.

Anhand des aufgezeichneten Problems und der aus dem Stand der Technik bekannten Lösungen, liegt der Erfindung die Aufgabe zugrunde, eine Bohrhilfe bereitzustellen, anhand der eine Führungsbohrung für ein Zahnimplantat relativ zu noch im Kiefer befindlichen Zähnen exakt gebohrt werden kann.

Erfindungsgemäß wird diese Aufgabe dadurch gelöst, daß bei einem Verfahren zur Herstellung einer Bohrhilfe für ein Zahnimplantat die nachfolgenden Verfahrensschritte durchlaufen werden:
- Das Herstellen von Röntgenaufnahmen des Kiefers und das Erzeugen eines entsprechenden Meßdatensatzes,
- die dreidimensionale optische Vermessung der sichtbaren Oberfläche von Kiefer und Zähnen und die Erzeugung eines entsprechenden Meßdatensatzes,
- der Korrelation der Meßdatensätze von Röntgenaufnahme und der Meßdatensätze der dreidimensionalen optischen Vermessung,
- der Planung des Implantattyps und der Implantatposition (Ort, Winkel) vorzugsweise anhand der Röntgendaten,
- dem Errechnen der Position (Ort, Winkel, Tiefe) des Implantatführungsloches relativ zu den erfaßten Oberflächen der Nachbarzähne,
- dem Erzeugen einer Bohrschablone, die die Negative der Oberflächen der Nachbarzähne und eine an vorbestimmter Stelle liegende Öffnung enthält.

Mittels des erfindungsgemäß vorgeschlagenen Verfahrens werden die Röntgenaufnahme und die tatsächlichen optischen Verhältnisse im Munde eines Patienten anhand einer Verknüpfung beider Aufnahmen so miteinander verbunden, daß eine Bohrhilfe in Form einer Bohrschablone zur Verfügung gestellt werden kann, die das Führungsloch an der für die Befestigung des Implantates anhand der Lage von Nachbarzähnen optimalen Position enthält. Der Zahnarzt folgt der durch die Bohrhilfe gegebenen Hilfestellung für die Führungsbohrung und geht somit sicher, nicht die im Kiefer verlaufenden Nervenstränge zu treffen, deren Lage zwar nicht aus der dreidimensionalen Oberflächenmessung hervorgeht, sondern aus der Röntgenaufnahme bekannt ist.

In weiterer Ausgestaltung des erfindungsgemäß vorgeschlagenen Verfahrens kann die Röntgenaufnahme eine Panoramaschichtaufnahme, eine tomosynthetische Aufnahme oder auch eine auf computertomographischem Wege erzeugte Aufnahme sein. Mittels der dreidimensionalen optischen Vermessung von sichtbaren Oberflächen werden bevorzugt die Okklusalflächen dem Implantat benachbarter noch vorhandener Zähne im Kiefer vermessen. Aus der Korrelation der Meßdatensätze der Röntgenaufnahme und der Meßdatensätze der dreidimensionalen optischen Aufnahme, sind die sichtbaren und für das menschliche Auge unsichtbaren - etwa der Nervenverlauf - Verhältnisse im Implantatbereich bekannt und erlauben das sichere Einbringen einer Führungsbohrung in den Kiefer.

Zur Korrelation der Röntgenaufnahme mit der dreidimensionalen optischen Aufnahme der sichtbaren Strukturen können Marker eingesetzt werden. Diese - beispielsweise als Kugeln ausgebildeten Körper - sind sowohl in der Röntgenaufnahme, als auch in der dreidimensionalen optischen Aufnahme des Kiefers sichtbar. Durch ein In-Deckung-Bringen der Marker läßt sich auf einfache Weise durch den Benutzer eine interaktive Korrelation der Röntgenaufnahme mit der dreidimensionalen optischen Aufnahme der sichtbaren Strukturen erzeugen.

Eine Korrelation der Meßdatensätze der Röntgenaufnahme und der dreidimensionalen optischen Aufnahme kann auch dadurch erfolgen, daß Meßdatensätze der dreidimensionalen optischen Aufnahme unter Annahme von Standardröntgenabsorptionswerten in Pseudoröntgenaufnahmen umgerechnet werden. Die tatsächliche Röntgenaufnahme und die Pseudoröntgenaufnahme lassen sich aus mehreren Richtungen betrachtet in Deckung bringen, beispielsweise anhand longitudinaler und transversaler Schnitte in der Panoramaröntgenaufnahme. Eine Korrelation kann auch erfolgen, indem zumindest teilweise aus den Röntgenaufnahmen die Oberflächenformen extrahiert werden, wie sie bei der optischen Aufnahme erfaßt werden und dann mit den Daten der optischen Aufnahme zur Deckung gebracht werden. Dies kann auf automatischem Wege oder auch interaktiv geschehen.

Anhand der Röntgendaten kann das Implantat in bekannter Weise bestimmt und positioniert werden. Anhand der gewonnenen Informationen über die Oberflächenstruktur, d. h. die Okklusalflächen benachbarter Zähne kann eine Implantationshilfe in Gestalt einer Bohrschablone an einer CAD/CAM-Einheit ausgeschliffen werden, wobei an der Bohrhilfe die Form der Okklusalflächen noch vorhandener Nachbarzähne im Negativ wiedergegeben sind. Die Bohrhilfe enthält eine Bohrung, die der Führung des Bohrers des Zahnarztes für die Bohrung zur Befestigung des Implantates dient. Die Negativformen der Oklusalflächen erlauben die eindeutige Positionierung der Bohrschablone im Munde des Patienten.

Anhand der Zeichnung wird die Erfindung nachstehend näher erläutert.

Es zeigt:
- Figur 1: Eine Panoramaschichtaufnahme in schematischer Form,
- Figur 2: die Erzeugung einer dreidimensionalen optischen Aufnahme von sichtbaren Strukturen im Kiefer,
- Figur 3: die Erzeugung einer Pseudoröntgenaufnahme aus einer dreidimensionalen Aufnahme sichtbarer Strukturen,
- Figur 4: die Superposition von Röntgenaufnahme und dreidimensionaler optischer Aufnahme oder Pseudoröntgenaufnahme B' und
- Figur 5: den für ein Implantat vorgesehenen Zwischenraum zwischen zwei benachbarten Zähnen und einer dort aufgenommenen Bohrhilfe.

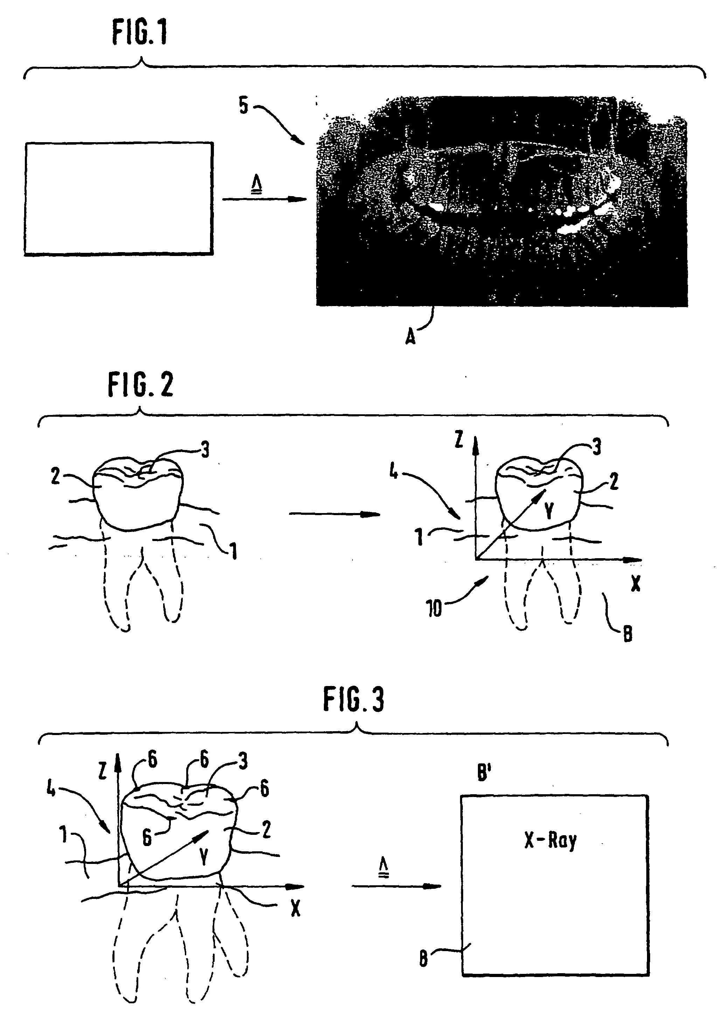

Fig. 1 zeigt eine stark schematisierte Röntgenaufnahme.

In der aufgezeigten Darstellung gemäß Fig. 1 ist als Röntgenaufnahme eine Panoramaschichtaufnahme dargestellt, die Ober- und Unterkieferbereich in einer ebenen Darstellung wiedergibt. Anstelle einer Panoramaschichtaufnahme ist auch eine tomosynthetische Röntgenaufnahme, wie auch eine computertomographische Röntgenaufnahme denkbar.

Fig. 2 zeigt schematisiert eine dreidimensionale optische Aufnahme eines Molaren 2, bei dem die Oberflächenstruktur, die Okklusalfläche 3 anhand eines dreidimensionalen Koordinatensystems 4 vermessen wird. Neben der Vermessung eines Molaren 2 wie hier dargestellt, kann ebensogut ein gesamter Kieferast vermessen werden, sei im Ober- oder im Unterkiefer. Die Röntgenaufnahme 5 bzw. die dreidimensionale optische Aufnahme 10 lassen sich als Meßdatensätze speichern und ablegen; die jeweiligen Meßdatensätze für die Aufnahme 5, 10 lassen sich anhand bestimmter Vorgabekriterien miteinander korrelieren.

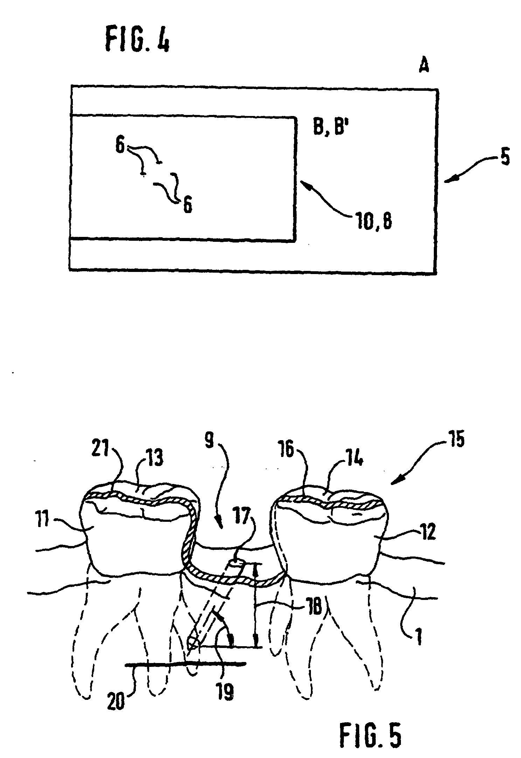

Durch die Korrelation, die auf unterschiedliche Weise erfolgen kann, werden die unsichtbaren, sich dem menschlichen Auge nicht darstellenden Gegebenheiten an der Implantatstelle 9, mit den für den Zahnarzt erkennbaren Gegebenheiten verknüpft. Die Korrelation kann einerseits durch Überlagerung einer Röntgenaufnahme A mit einer dreidimensionalen Aufnahme B erfolgen (vgl. Fig. 5), und anhand von Markern 6, befestigt an den Zähnen 2, Fixpunkte erzeugt werden, wodurch eine Röntgenaufnahme A und eine dreidimensionale B miteinander in Deckung gebracht werden können und eine Korrelation der beiden Meßdatensätze erfolgen kann. Anstelle von Markern 6, die an den Zähnen 2 befestigbar sind, kann aus den Oberflächendaten der dreidimensionalen Aufnahme 10 unter Annahme von Standard-Röntgenabsorptionswerten und der Entstehungstheorie des jeweiligen Röntgenbildes eine Pseudoröntgenaufnahme B', 8 erzeugt werden. Die vorliegenden Röntgenaufnahme 5 und die Pseudoröntgenaufnahme 8 können vom Benutzer in einem interaktiven Schritt in Deckung miteinander gebracht werden. Führt der Benutzer die Korrelation aus mehreren Richtungen durch, ist die Korrelation der Röntgenaufnahme 5 mit der dreidimensionalen Aufnahme 10 voll bestimmt. Wird als Röntgenaufnahme eine Panoramaschichtaufnahme gewählt, so kann der Benutzer dies mit longitudinal und transversal verlaufenden Schnitten durch die Panoramaschichtaufnahme erreichen. Damit wären X- und Y-Richtung eines Koordinatensystems 4 abgedeckt.

In der Röntgenaufnahme 5 gemäß Fig. 1 wird die Position des Implantates geplant, wobei seine Position im Kiefer 1 auf einer, in der Regel jedoch aus zwei Richtungen gesehen, festgelegt wird. Aus der Röntgenaufnahme A, Bezugszeichen 5, ist der Verlauf des Nerves 20 im unteren Bereich des Kiefers 1 bekannt, anhand dessen Lage die Bohrlochtiefe 18 (vergleiche Fig. 5) geplant werden kann. Die Korrelation der Röntgenaufnahme 5 mit der dreidimensionalen optischen Aufnahme 10 erlaubt ein Einbeziehen der der Implantatposition 9 benachbart angeordneten Zähne 11 und 12. Deren Positionen sind sowohl aus der Röntgenaufnahme 5 als auch aus der dreidimensionalen optischen Aufnahme 10 bekannt. Aus der dreidimensionalen optischen Aufnahme 10 resultieren zusätzlich noch die Okklusal-Oberflächenstrukturen 13 und 14 der benachbarten Zähne 11 und 12. Da die Röntgenaufnahme und die 3D-Aufnahme zueinander korreliert sind, ist die Lage des geplanten Implantates relativ zu den Oklusalflächen der Nachbarzähne bekannt.

Somit kann auch der Ort der Bohrung und die Bohrtiefe bestimmt werden.

Der Benutzer kann nun durch Eingabe von Linienzügen festlegen, wie die Größe der Bohrschablone beschaffen sein soll. Anhand der Meßdaten ist eine CAD/CAM-Maschine in der Lage, die Bohrschablone mit dem Negativ der Oklusalflächen und Führungslauf für den Bohrer zu fertigen.

Eine Fixierung einer solcher Art erhaltenen Bohrhilfe 16 erfolgt mittels einer lösbaren Haftschicht 21 auf den Okklusalflächen 13 und 14 benachbarte Zähne 11 und 12, die die Implantatposition 9 begrenzen. Die Bohrhilfe 16 oder auch Bohrschablone kann in vorteilhafter Weise auf einer Präzisionswerkzeugmaschine, die dreidimensional betrieben werden kann, gefertigt werden, auf der sich auch Zahnersatz herstellen läßt.

Die exakte Positionierung der Bohrhilfe 16 in Bezug auf den Kieferabschnitt 15 der Implantatposition 9 wird dadurch erzielt, daß die Negativform der Bohrhilfe 16 auf die Okklusalflächen 13, 14 der benachbarten Zähne 11 und 12 aufsetzt. Die Position des Führungsloches 17 auf der Oberfläche der Bohrhilfe 16 vorgegeben, ebenso die Neigung 19 in der er den Bohrer anzusetzen hat. Die Bohrtiefe 18 ist dem Zahnarzt aus der Korrelation der Meßdatensätzen von Röntgenaufnahme 5, sei es als Panoramaschichtaufnahme gehalten oder als eine tomosynthetische Aufnahme vorliegend, bestimmt und wird auf die Bohrschablone als Anschlag übertragen. Da sich auf der Oberseite der Bohrhilfe 16 die Position des Führungsloches 17 befindet, kann der Zahnarzt die Bohrung im Kiefer 1 vornehmen mit der Sicherheit, die für die Befestigung des Implantates zwischen den Nachbarzähnen 11 und 12 optimale Führungslochposition gewählt zu haben.

### Bezugszeichenliste

- 1.: Kiefer
- 2.: Zahn
- 3.: Okklusalfläche
- 4.: Koordinatensystem
- 5.: Röntgenaufnahme
- 6.: Marker
- 7.: Superposition
- 8.: Pseudo-Röntgenaufnahme
- 9.: Implantatposition
- 10.: 3D-Aufnahme
- 11.: benachbarter Zahn
- 12.: benachbarter Zahn
- 13.: Okklusalflächen
- 14.: Okklusalflächen
- 15.: Kieferabschnitt
- 16.: Bohrhilfe
- 17.: Führungslochposition
- 18.: Bohrtiefe
- 19.: Bohrungsneigung
- 20.: Nerv
- 21.: Haftschicht

- A: Röntgenaufnahme
- B: 3D-Aufnahme
- B': Pseudoröntgenaufnahme

## Patentansprüche

1. Verfahren zur Erstellung einer Bohrhilfe (16) für ein Zahnimplantat mit nachfolgenden Verfahrensschritten:
• dem Verwenden von Röntgenaufnahmen (5) des Kiefers (1) zur Erzeugung eines entsprechenden Messdatensatzes,
• der Möglichkeit zur Bestimmung eines optimalen Bohrlochs für ein Implantat vorzugsweise aufgrund der Röntgenaufnahme,
• der Möglichkeit zur Bestimmung eines Führungslochs in einer Bohrschablone (16), **gekennzeichnet durch**
• die dreidimensionale optische Vermessung der sichtbaren Oberfläche von Kiefer (1) und Zähnen (2) und Erzeugen eines entsprechenden Messdatensatzes,
• die Korrelation der Messdatensätze der Röntgenaufnahme (5) und der Messdatensätze der dreidimensionalen optischen Vermessung (10) und
• der Möglichkeit zur Bestimmung eines Führungslochs in einer Bohrschablone (16) relativ zu den Oberflächen der Nachbarzähne aufgrund von Röntgenaufnahmen und optischer Vermessung.

2. Verfahren gemäß Anspruch 1, **dadurch gekennzeichnet, daß** die Röntgenaufnahme (5) Panoramaschichtaufnahmen, tomosynthetische Aufnahme oder computertomographsiche Aufnahmen sind.

3. Verfahren gemäß Anspruch 1, **dadurch gekennzeichnet, daß** die dreidimensional vermessene, sichtbare Oberfläche (13, 14) die Okklusalflächen benachbarter Zähne (11, 12) am Kiefer (1) sind.

4. Verfahren gemäß Anspruch 1, **dadurch gekennzeichnet, daß** die Korrelation der Maßdatensätze von Röntgenaufnahme (5) und dreidimensionaler optischer Aufnahme (10) anhand von auf Zähnen (2) aufgebrachter Marker (6) erfolgt.

5. Verfahren gemäß Anspruch 4, **dadurch gekennzeichnet, daß** die Marker (6) Kugeln sind.

6. Verfahren gemäß Anspruch 1, **dadurch gekennzeichnet, daß** die Meßdatensätze der dreidimensionalen optischen Vermessung und unter Zugrundelegung von Standard-Röntgenabsorptionswerten und der Entstehungstheorie des jeweiligen Röntgenbildes in eine Pseudoröntgenaufnahme (8) umgerechnet werden.

7. Verfahren gemäß der Ansprüche 1 und 6, **dadurch gekennzeichnet, daß** die Röntgenaufnahme (5) und die Pseudoröntgenaufnahme (8) aus mehreren Richtungen zur Deckung gebracht werden.

8. Verfahren gemäß Anspruch 7, **dadurch gekennzeichnet, daß** es sich bei der Röntgenaufnahme (5) um mindestens zwei Einzelpanoramaaufnahmen handelt, die longitudinale und transversale Schnitte durch den Kiefer zeigen.

9. Verfahren gemäß Anspruch 1, **dadurch gekennzeichnet, daß** die Bohrhilfe (16) auf einem dimensionsstabilen Material ausgeschliffen wird, wobei dieses die Form der Okklusalflächen (13, 14) von Nachbarzähnen (11, 12) der Implantierungsposition (9) im Negativ wiedergibt.

10. Verfahren gemäß Anspruch 9, **dadurch gekennzeichnet, daß** die Bohrhilfe (16) eine Bohrungsposition (17) enthält, die zur Führung des Bohrers dient.

## Claims

1. Method for creating a drill aid (16) for a tooth implant, with the following method steps:
- the use of X-ray images (5) of the jaw (1) to generate a corresponding set of measurement data,
- the possibility of determining an optimal drill hole for an implant, preferably based on the X-ray image,
- the possibility of determining a guide hole in a drill template (16), **characterized by**
- the three-dimensional optical measurement of the visible surface of jaw (1) and teeth (2) and the generation of a corresponding set of measurement data,
- the correlation of the sets of measurement data from the X-ray image (5) and of the sets of measurement data from the three-dimensional optical measurement (10), and
- the possibility of determining a guide hole in a drill template (16) relative to the surfaces of the neighbouring teeth based on X-ray images and optical measurement.

2. Method according to Claim 1, **characterized in that** the X-ray images (5) are panoramic tomograms, tomosynthetic images or computed tomography images.

3. Method according to Claim 1, **characterized in that** the three-dimensionally measured, visible surfaces (13, 14) are the occlusal surfaces of neighbouring teeth (11, 12) on the jaw (1).

4. Method according to Claim 1, **characterized in that** the sets of measurement data from the X-ray image (5) and those from the three-dimensional optical image (10) are correlated using markers (6) attached to teeth (2).

5. Method according to Claim 4, **characterized in that** the markers (6) are balls.

6. Method according to Claim 1, **characterized in that** the sets of measurement data from the three-dimensional optical measurement are converted to a pseudo X-ray image (8) on the basis of standard X-ray absorption values and the generation theory of the respective X-ray image.

7. Method according to Claims 1 and 6, **characterized in that** the X-ray image (5) and the pseudo X-ray image (8) are brought into superposition from several directions.

8. Method according to Claim 7, **characterized in that** the X-ray image (5) involves at least two individual panoramic images showing longitudinal and transverse sections through the jaw.

9. Method according to Claim 1, **characterized in that** the drill aid (16) is ground out from a dimensionally stable material, the latter representing the form of the occlusal surfaces (13, 14) of neighbouring teeth (11, 12) of the implantation position (9) as a negative.

10. Method according to Claim 9, **characterized in that** the drill aid (16) contains a drilling position (17) that serves to guide the drill.

## Revendications

1. Procédé de création d'un dispositif de perçage (16) d'un implant dentaire, comportant les étapes des procédé suivantes:
- l'utilisation de radiographies (5) de la mâchoire (1) afin de produire une séquence de données de mesure correspondante,
- la possibilité de déterminer un trou percé optimal pour un implant, de préférence en se basant sur la radiographie,
- la possibilité de déterminer un trou d'introduction dans un gabarit de perçage (16), **caractérisé par**
- le relevé optique en trois dimensions de la surface visible de la mâchoire (1) et des dents (2) et la production d'une séquence de données de mesure correspondante,
- la corrélation des séquences de données de mesure de la radiographie (5) et des séquences de données de mesure du relevé (10) optique en trois dimensions et
- la possibilité de déterminer un trou d'introduction dans un gabarit de perçage (16) relativement aux surfaces des dents voisines en se basant sur des radiographies et un relevé optique.

2. Procédé selon la revendication 1, **caractérisé en ce que** les radiographies (5) sont des radiographies panoramiques, par tomosynthèse ou par tomographie informatique.

3. Procédé selon la revendication 1, **caractérisé en ce que** les surfaces visibles (13, 14), relevées en trois dimensions sont des dents (11, 12) voisines des surfaces occlusales sur la mâchoire (1).

4. Procédé selon la revendication 1, **caractérisé en ce que** la corrélation des séquences de données de mesure de la radiographie (5) et du relevé optique en trois dimensions (10) est effectuée en se basant sur des marqueurs (6) appliqués sur les dents (2).

5. Procédé selon la revendication 4, **caractérisé en ce que** les marqueurs (6) sont des billes.

6. Procédé selon la revendication 1, **caractérisé en ce que** les séquences de données de mesure du relevé optique en trois dimensions sont converties en une pseudo radiographie en prenant pour base les valeurs standard d'absorption des rayons X et la théorie de génération de l'image radiographique (8) respective.

7. Procédé selon les revendications 1 et 6, **caractérisé en ce que** la radiographie (5) et la pseudo radiographie (8) sont amenées à se superposer à partir de plusieurs directions.

8. Procédé selon la revendication 7, **caractérisé en ce que** la radiographie (5) désigne au moins deux radiographies à panorama individuel, qui représentent des coupes longitudinales et transversales à travers la mâchoire.

9. Procédé selon la revendication 1, **caractérisé en ce que** le dispositif de perçage (16) est meulé sur un matériau aux dimensions stables, moyennant quoi ce dernier rend en négatif la forme des surfaces occlusales (13, 14) des dents voisines (11, 12) de la position d'implantation (9).

10. Procédé selon la revendication 9, **caractérisé en ce que** le dispositif de perçage (16) contient une position de perçage (17), qui sert à introduire le foret.
